# EUROPEAN PATENT APPLICATION

(11) **EP 1 148 039 A1**
(43) Date of publication of application: **24.10.2001**
(21) Application number: 01108597.4
(22) Date of filing: 05.04.2001
(51) Int. Cl.: C07C 17/37, C07C 19/08

(54) **A process for the production of CHF3 (HFC-23)**

(30) Priority: 21.04.2000 IT MI000903
(71) Applicant: Ausimont S.p.A., 20121 Milano (IT)
(72) Inventor: Cuzzato, Paolo, 31050 Ponzano Veneto, Treviso (IT)
(74) Representative: Sama, Daniele, Dr.

(57) **Abstract**

A high yield process for the production of CHF₃ (HFC-23) by gaseous phase dismutation of CHClF₂ (HCFC-22) on a heterogeneous catalyst.

## Description

The present invention relates to a process for the production of CHF₃ (HFC-23) with a high productivity.

Specifically, the invention relates to a high yield process for the CHF₃ (HFC-23) synthesis, obtained starting from CHClF₂ (HCFC-22) by using a specific heterogeneous catalyst.

The need to have available efficient processes for the HFC-23 synthesis is known in the prior art. HFC-23 is a hydrofluorocarbon used as a refrigerant for replacing some chlorofluorocarbons dangerous to the stratospheric ozone. In fact, HFC-23, being free from chlorine atoms, has no dangerous action on the atmospheric ozone, as required by the Montreal protocol and subsequent amendments. Another important application of HFC-23 is as flame-extinguishant as substitute of HALONS®, see patent application WO 91/04766.

Processes for the preparation of HFC-23 by fluorination with anhydrous HF of HCFC-22 or of its precursors (in particular chloroform CHCl₃ or methane CH₄) are known. Said processes generally use as catalyst compounds based on antimony halides. An example of such processes is found in USP 4,138,355 wherein the production of trifluoromethane HFC-23 starting from chloroform CHCl₃, by reacting said compound with liquid HF in the presence of an equimolar mixture of antimony pentahalides and antimony trihalides, is mentioned. The drawback of these processes is to use toxic and corrosive reactants, such as hydrofluoric acid, elemental chlorine, and as a catalyst a compound based on a toxic metal. Besides, there are hard reaction conditions, which lower the process selectivity and increase the plant wear and the energetic costs.

The need was therefore felt to produce HFC-23 with a high yield and productivity by a simple, cheap, safe process, overcoming the above mentioned drawbacks of the prior art.

An object of the present invention is a process for the production of CHF₃ (HEC-23) by gaseous phase dismutation of CHClF₂ (HCFC-22) on a heterogeneous catalyst selected from aluminum fluoride AlF₃; oxides, halides, oxy-halides of trivalent chromium, optionally supported.

The conditions under which the reaction advantageously takes place are:
- reaction temperture in the range 50°-300°C, preferably 90°-200°C;
- contact time in the range 1-30 seconds, preferably 1-10 seconds.

The pressure is not critical: one commonly operates at atmospheric or higher pressure.

The catalyst for promoting the dismutation object of the present invention can be aluminum fluoride AlF₃, optionally modified by the addition of small amounts (generally lower than 1% by weight with respect to AlF₃) of transition metals, preferably Fe, Ni, Co, Mn. Such a catalyst is for example described in USP 5,600,037 in the name of the Applicant. AlF₃ has a fluorine content of at least 90% of the stoichiometric value, preferably not lower than 95%. The aluminum fluoride is preferably in the crystallographic gamma form, optionally in mixture with the beta or delta form, as described in the patent FR 1,383,927. Preferably AlF₃ has a surface area comprised between 15 and 40 m²/g (as determined through B.E.T.).

Alternatively, as a catalyst, oxides, halides, oxy-halides of trivalent chromium can be used. Such chromium compounds can be used as such, or, preferably, supported on AlF₃ as above defined. See for example USP 5,414,167 in the name of the Applicant.

The dismutation process of HCFC-22 of the present invention takes place in a continuous way, both in a fixed and fluidized bed reactor; in the latter case the catalyst must have a suitable size.

In the preferred embodiment of the invention, the reactant HCFC-22 is let continuously flow on a catalyst bed maintained at the preferred above temperature, and the product HFC-23 is separated from the unconverted reactant and from optional intermediates and by-products by methods known in the prior art, usually by fractionated distillation.

The Applicant has surprisingly and unexpectedly found that under the above mentioned preferred conditions, the yield of HFC-23 is very high, up to 99% of the theoretic value (see the Examples) and the amount of intermediates (specifically HCFC-21, CHFCl₂) is very low, up to less than 1% by moles, wherefore their recovery is industrially not very interesting. Furthermore the reactant conversion of HCFC-22 is generally very high wherefore there is no need to recycle the unreacted starting compound. This is a further advantage of the invention process, since both the investment necessary to the plant construction and the energetic cost of its running are much reduced. Also the low temperature at which the reaction can be carried out helps in limiting the latter.

Another advantage of the present invention is the very high process selectivity, wherefore on the one hand there is no formation of by-products to be disposed of, on the other hand the product HFC-23 obtained after distillation shows a very good purity.

The reactant can be fed pure or diluted with a gas inert under the reaction conditions, for example helium or nitrogen.

The catalyst is prepared by one of the methods known in the prior art, among them the Applicant has found particularly advantageous:
- for the catalyst based on aluminum fluoride, the fluorination by anhydrous HF of hydrated aluminum oxide (alumina), preferably in the crystalline form of pseudo-bohemite and optionally additived with small amounts, generally from 1 to 15% by weight, of silica. The fluorination is continued until having a fluorine content not lower than 90%, preferably 95%, of the stoichiometric value with respect to AlF₃;
- for the catalyst based on chromium, the impregnation of a soluble chromium salt on a support formed by aluminum fluoride as above defined, by the method commonly known in the prior art as incipient wetness (dry impregnation). On the basis of this method the impregnation is carried out by pouring, in sequence, on the AlF₃ support aliquots of an impregnating solution, such that the volume of each aliquot is not higher than the volume of the aluminum fluoride pores. The impregnating solution is prepared by dissolving in water the required amounts of the corresponding salts, preferably chlorides, of the trivalent chromium. The solution is poured in aliquots on the support, drying at 110°C for some hours after each addition, to evaporate the water from the support pores. The chromium amount in the supported catalyst is generally in the range 5-15% by weight, preferably 10-15% by weight, calculated as chromium amount on the weight of the supported catalyst.

When the catalyst is not supported, it is prepared by methods known in the prior art, for example by precipitation of a soluble trivalent chromium salt as described in USP 5,345,014.

In the process of the present invention, the catalyst is preferably dried before the starting of the reaction by heating at 300°-400°C in inert gas flow, optionally followed by a treatment with anhydrous (pure or diluted) HF at the same temperature.

The present invention will be better illustrated by the following Examples, which have a merely indicative but not limitative purpose of the scope of the invention itself.

### EXAMPLES

### Characterization

The HCFC-22 fed as reactant in Examples 1-5 is produced by the Applicant itself and it has a purity equal to or higher than 99.9%, containing only some hundreds of ppm of CH₂F₂ (HFC-32), CF₂Cl₂ (CFC-12) and CHFCl₂ (HCFC-21) .

In all the Examples the flow-rates of the gases fed into the reactor are meant measured at atmospheric pressure and at the temperature of 20°C.

The aluminum fluoride used as catalyst in Examples 1-3, and as a support in Examples 4-5, has been prepared by fluorination of a commercial alumina containing silica (Akzo M® or Condea Siral®).

The catalyst of Examples 4-5 has been prepard by dry impregnation of an aqueous CrCl₃ solution an AlF₃ according to the method mentioned in the description and it has a chromium content of about 11% by weight.

In the Examples the terms of conversion, yield, productivity, nominal contact time are used with the following meaning:
- CONVERSION = Ratio between the converted reactant moles and the starting reactant moles;
- YIELD = Ratio between the obtained moles of an useful product and the total converted reactant moles;
- PRODUCTIVITY = Amount by weight of the useful product obtained for unit of time and unit of weight of the used catalyst;
- NOMINAL CONTACT TIME = ratio between the volume of the fed gases and the volume of the catalytic bed, at the reaction temperature and at atmospheric pressure.

### EXAMPLE 1

In a tubular reactor having a 7 mm diameter, electrically heated and equipped with porous septum at the base, 1.0 g of an aluminum fluoride are introduced, prepared by fluorination of a commercial alumina containing silica (Akzo M® or Condea Siral® ) until obtaining the fluorine content as indicated in the description.

After a pretreatment with nitrogen and subsequently with a nitrogen/HF mixture in molar ratio 1:1 at the temperature of 360°C, a mixture formed by 12 cc/min of helium and 21.6 cc/min of the reactant HCFC-22 is fed into the reactor, at a pressure slightly higher than the atmospheric one and at the temperature of 100°C.

The nominal contact time is equal to 1.3 seconds. The gases coming out from the reactor are analyzed by GLC with a thermoconductivity detector and they result to contain in % by moles:

| | |
|---|---|
| HFC-23 | 54.2 |
| HCFC-22 | 17.3 |
| HCFC-21 | 2.7 |
| CHCl₃ (HCC-20) | 25.8 |
| Others | negligible |

The reactant HCFC-22 conversion is equal to 82.7%.
The HFC-23 yield (ratio between the produced HFC-23 and the converted HCFC-22) is of 65.6%, i.e. equal to 98% of the theoretic maximum (the theoretic maximum is given by the stoichiometric yield of the reaction: 3 CHClF₂ → 2 CHF₃ + CHCl₃, wherefore it results 2/3 of the converted HCFC-22, i.e. 66.7%).

The productivity in HFC-23, expressed as weight of the produced HFC-23 per unit of weight of the catalyst and per hour, is equal to about 2,000 g/Kg_{cat} x h. Within the sensitivity of the used analysis method, products different from the halomethanes of formula CHX₃ with X=F or Cl, cannot be quantified.

### EXAMPLE 2

Example 1 is repeated, using the same amount of catalyst and of reactant HCFC-22, with the only change that the reaction temperature is brought to 200°C.

The HCFC-22 conversion increases to 97% and the net HCF-23 yield is 65.9%. The HFC-23 productivity in this case increases to about 2,500 g/Kg_{cat} x h.

### EXAMPLE 3

3.5 g of the same AlF₃ are introduced in the same reactor used in Example 1, and they are activated as above described. 24 cc of HCFC-22 are then fed, without any diluent, at the temperature of 100°C, thus obtaining a nominal contact time equal to 6.3 seconds.

The gases coming out from the reactor are analyzed by GLC with a thermoconductivity detector and they result to contain in % by moles:

| | |
|---|---|
| HFC-23 | 65.7 |
| HCFC-22 | 1.1 |
| HCFC-21 | 0.9 |
| HCC-20 | 32.4 |
| Others | negligible |

The reactant conversion is equal to 98.9% and the HFC-23 yield is 66.4%, almost equal to the theoretic maximum. The HFC-23 productivity is equal to about 800 g/Kg_{cat} x h. Within the sensitivity of the used analysis method, products different from the halomethanes of formula CHX₃ with X=F or Cl, are not evident.

### EXAMPLE 4

1.0 g of a catalyst, which is a compound of chromium supported on AlF₃, prepared by dry impregnation of AlF₃ with an aqueous solution of CrCl₃, having a chromium content of about 11% by weight, are introduced into the same reactor of the previous Examples, after a pretreatment with nitrogen and HF.

19.7 cc/min of HCFC-22, diluted with 14 cc/min of helium, are then fed at the temperature of 100°C, thus obtaining a nominal contact time equal to 1.1 seconds.

The gases coming out from the reactor are analyzed by GLC with a thermoconductivity detector and they result to contain in % by moles:

| | |
|---|---|
| HFC-23 | 57.7 |
| HCFC-22 | 12.3 |
| HCFC-21 | 2.4 |
| CHCl₃ | 27.6 |
| Others | negligible |

The reactant conversion is equal to 87.7% and the HFC-23 yield is 65.8%, equal to almost 99% of the theoretic maximum. The HFC-23 productivity is equal to almost 2,000 g/Kg_{cat} x h. Within the sensitivity of the used analysis method, products different from the halomethanes of formula CHX₃ with X=F or Cl, are not evident.

### EXAMPLE 5

All the other conditions of Example 4 remaining the same, the temperature is brought to 200°C. The HCFC-22 conversion increases up to 97% and the HFC-23 yield is almost equal to the theoretic maximum. The HFC-23 productivity increases to almost 2,300 g/Kg_{cat} x h.

As it can be seen from the Examples, both the temperature and the contact time have effect on the HCFC-22 conversion.

Since the analysis with a thermoconductivity detector (TCD) is not sufficient to put in evidence the compounds present in traces, some analyses are repeated by GC-MS (mass spectrometry) on the reaction product.

Both under the conditions of Example 3 and under those of Examples 4-5, few hundreds ppm of by-products are found, represented by CF₃Cl, C₂ClF₅ and CHCl₂, among which the only potential contaminating agent of the product, on the basis of the boiling points, is CF₃Cl (CFC-13), present in the range of about one hundred ppm. This compound is formed almost certainly from CFC-12 present as an impurity in the reactant. This analysis confirms that the invention process selectivity is almost unitary.

## Claims

1. A process for the production of CHF₃ (HFC-23) by gaseous phase dismutation of CHClF₂ (HCFC-22) on a heterogeneous catalyst selected from:
- aluminum fluoride AlF₃;
- oxides, halides, oxy-halides of trivalent chromium.

2. A process according to claim 1, wherein:
- the reaction temperature is in the range 50°-300°C;
- the contact time is in the range 1-30 seconds.

3. A process according to claim 2, wherein:
- the reaction temperature is in the range 90°-150°C;
- the contact time is in the range 1-10 seconds.

4. A process according to claims 1-3, wherein the catalyst AlF₃ is modified by the addition of an amount lower than 1% by weight with respect to AlF₃, of transition metals selected from Fe, Ni, Co, Mn.

5. A process according to claims 1-4, wherein AlF₃ has a fluorine content of at least 90% of the stoichiometric value, preferably not lower than 95%, and it is preferably under the crystallographic gamma form.

6. A process according to claims 1-5, wherein the oxides, halides, oxy-halides of trivalent chromium are supported on AlF₃.

7. A process according to claims 1-6, whereien the catalyst is dried before the starting of the reaction by heating at 300°-400°C in inert gas flow, optionally followed by a treatment with anhydrous HF at the same temperature.
